# EUROPEAN PATENT APPLICATION

(11) **EP 1 815 815 A1**
(43) Date of publication of application: **08.08.2007**
(21) Application number: 07250438.4
(22) Date of filing: 02.02.2007
(51) Int. Cl.: A61B 19/00, A61B 10/02, A61B 17/34

(54) **Guided disposable fiducial for breast biopsy localization fixture**

(30) Priority: 03.02.2006 US 346715
(71) Applicant: ETHICON ENDO-SURGERY, INC., Cincinnati, Ohio 45242 (US)
(72) Inventor: Dietz, Timothy G., Terrace Park, OH 45174 (US)
(74) Representative: Tunstall, Christopher Stephen

(57) **Abstract**

A fiducial device enhances shelf life and clinical flexibility by including an elongate cavity of a fiducial pointer that may be filled with an imageable material immediately prior to use. A fiducial holder of the fiducial device is guided by a targeting rail of a breast coil localization fixture to a desired position external to a patient. Alternatively, a sleeve guided by the targeting rail may receive the fiducial pointer for providing an internal imageable target.

## Description

### Cross Reference to Related Applications

The present application is a continuation in part of U.S. Pat. Appln. Ser. No. 11/103,959, "MRI BIOPSY DEVICE LOCALIZATION FIXTURE" to Hughes et al., filed on 12 April 2005, the disclosure of which is hereby incorporated by reference in its entirety.

### Field of the Invention

The present invention relates, in general, to a method of imaging assisted tissue sampling and, more particularly, to an improved method for positioning a biopsy probe with respect to a magnetic resonance imaging (MRI) breast coil for acquiring subcutaneous biopsies and for removing lesions.

### Background of the Invention

Recently, core biopsy devices have been combined with imaging technology to better target a lesion in breast tissue. One such commercially available product is marketed under the trademark name MAMMOTOME^{™}, by Ethicon Endo-Surgery, Inc. An embodiment of such a device is described in U.S. Patent No. 5,526,822 issued to Burbank, et al., on June 18, 1996, and is hereby incorporated herein by reference. Its handle receives mechanical and electrical power as well as vacuum assist from a remotely positioned control module that is spaced away from the high magnetic field of a Magnetic Resonance Imaging (MRI) machine.

As seen from that reference, the instrument is a type of image-guided, percutaneous coring, breast biopsy instrument. It is vacuum-assisted, and some of the steps for retrieving the tissue samples have been automated. The physician uses this device to capture "actively" (using the vacuum) the tissue prior to severing it from the body. This allows the sampling of tissues of varying hardness. In addition, a side opening aperture is used, avoiding having to thrust into a lesion, which may tend to push the mass away, cause a track metastasis, or cause a haematoma that, with residual contrast agent circulating therein, may mimic enhancement in a suspicious lesion. The side aperture may be rotated about a longitudinal axis of the probe, thereby allowing multiple tissue samples without having to otherwise reposition the probe. These features allow for substantial sampling of large lesions and complete removal of small ones.

In Pub. No. US 2003/0199785 to Hibner et al., which is hereby incorporated by reference in its entirety, localization fixtures are described that are attachable to a breast coil. These localization fixtures aided in accurately positioning the probe to a location of a suspicious lesion within breast tissue. In particular, the X-Y-Z Cartesian coordinates of a suspicious lesion are referenced to a fiducial device inserted into a corner of a compression plate of the localization fixture through which the probe is inserted. Humanly visible measurement guides for each axis then allow the probe to be correspondingly positioned after a patient has been withdrawn from a closed bore MRI machine without the need for imaging the probe during insertion, referencing the coordinate information based on the external fiducial device.

While incorporating an external fiducial device into a localization fixture has a number of advantages in placing a core biopsy probe, a significant need exists for additional diagnostic and clinical flexibility in guiding a core biopsy probe with reference to an external fiducial device.

### Brief Summary of the Invention

The invention overcomes the above-noted and other deficiencies of the prior art by providing a fiducial device that includes an elongate cavity that may be filled with an imageable material immediately prior to use. Thereby the fiducial device advantageously has increased options for providing sterile imageable material to the user, enhanced shelf life and less rigorous packaging and storage constraints as well as allowing clinical flexibility in selecting a desired imageable, filling material.

In particular, the invention provides an apparatus for performing a minimally invasive medical procedure with reference to a diagnostic image taken of a patient's breast compressed between a medial compression member and a lateral compression member having a biopsy probe support positionable relative to the lateral compression member, the apparatus comprising: a housing formed of a magnetic resonance imaging (MRI) compatible material defining an internal cavity; a port formed in the housing communicating with the internal cavity and operatively configured to receive MRI imageable material; and a vent formed in the housing for allowing air evacuation during filling.

The port may comprise a septum or a one-way valve. It may comprise a two-way valve. It may comprises a leur fitting.

The housing may include an elongate portion and a proximal portion including an engagement mechanism operatively configured for engagement to the biopsy probe support.

The housing may comprises clear polycarbonate or an optically transmissive thermoplastic.

The vent may comprise a small hole or a porous plug, for example formed from a material selected from a group consisting of porous PTFE, porous polyethylene, porous polypropylene, porous polystyrene, and glass frit.

The housing may include an elongate portion shaped for insertion into a probe sleeve.

The invention further provides an apparatus for performing a biopsy with reference to a diagnostic image taken of a patient's breast, the apparatus comprising: a medial compression member; a lateral compression member moved relative to the medial compression member to compress and locate the patient's breast; a pedestal member positionably coupled to the lateral compression member for locating a lateral coordinate; and a targeting rail positionably coupled to the pedestal for locating a vertical coordinate and including a biopsy guide defining an angle of penetration.

The biopsy guide may further comprise a home position reference, the apparatus further comprising a fiducial holder attachable to the biopsy guide at the home position reference and laterally sized to align a fiducial proximate to the patient's breast at an insertion point along an axis of penetration of the biopsy instrument.

The fiducial holder may further comprise an integral fiducial comprising a magnetic resonance imaging (MRI) imageable material.

The fiducial holder may further comprise a polymer spring operatively configured to engage the biopsy guide.

The fiducial holder may further comprise an engagement member operatively configured to slidingly engage the biopsy guide to the home position reference.

The invention further provides an apparatus for performing a biopsy with reference to a diagnostic image taken of a patient's breast, the apparatus comprising: a first compression member positionable on a selected side of the patient's breast; a second compression member positionable on an opposite side of the patient's breast and moved relative to the first compression member to compress and locate the patient's breast; an opening formed in the first compression member; and a fiducial container fillable with an imageable fluid and sized for insertion into the opening in the first compression member.

The opening may comprise a selected one of a plurality of grid openings shaped for insertion of a probe of a core biopsy device.

The apparatus may further comprise a probe guide engageable to the first compression member and sized to receive a probe of a core biopsy device, the fiducial container comprising an elongate cannula shaped for insertion through the probe guide.

The probe guide may comprise a sleeve having a distal opening, the fiducial container comprises an introducer obturator including a distal piercing tip.

Objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIGURE 1 is a perspective disassembled view of a Magnetic Resonance Imaging (MRI) Biopsy System including a fiducial device consistent with aspects of the invention.

FIGURE 2 is a perspective view of an alternative pedestal and targeting rail supported by a lateral fence of a localization fixture for the MRI biopsy system of FIG. 1.

FIGURE 3 is a disassembled perspective view of an alternative guidance system for the pedestal and targeting rail of FIG. 2.

FIGURE 4 is proximal perspective view of a fiducial holder of the fiducial device of FIG. 1.

FIGURE 5 is a top view of the fiducial holder of FIG. 4.

FIGURE 6 is a proximal side view in elevation of the fiducial holder of FIG. 4.

FIGURE 7 is a right side view in elevation of the fiducial holder of FIG. 4.

FIGURE 8 is a top diagrammatic view of a disposable fiducial pointer of the fiducial device of FIG. 1.

FIGURE 9 is a top diagrammatic view of an alternate disposable fiducial pointer for the fiducial device of FIG. 1.

FIGURE 10 is a perspective view of a honeycomb lateral plate with an integral distal targeting fixture shown in its swung open position;

FIGURE 11 is a perspective view of the honeycomb lateral plate with the integral distal targeting fixture of FIG. 11, shown in its swung closed position with a probe guide installed with three fiducial devices;

### Detailed Description of the Invention

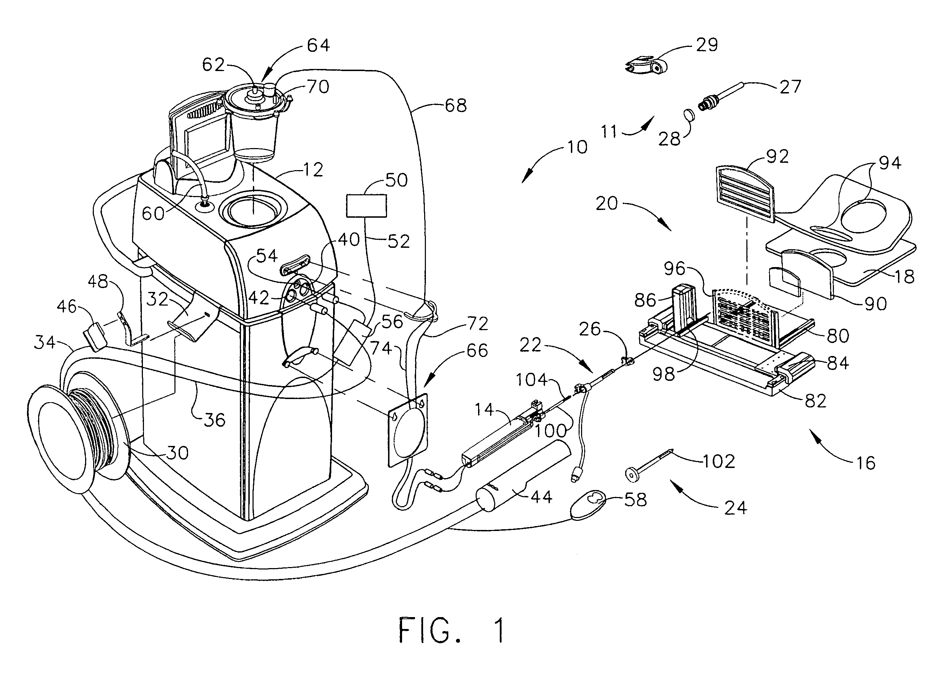

Turning to the Drawings, wherein like numerals denote like components throughout the several views, in FIG. 1, an Magnetic Resonance Imaging (MRI) compatible biopsy system 10 includes a fiducial device 11 (shown enlarged in foreground) to a desired position relative to a patient's breast so that coordinate information from an imaging scan may be fully exploited during subsequent taking of a core biopsy or radiological treatment. Clinical flexibility is enhanced by having the fiducial device 11 packaged without being filled with an imageable compound (e.g., saline, gadolinium solution). Thereby, the shelf life is increased, packaging and environmental constraints are less strenuous, and a wider range of imageable compounds / liquids may be selected by the user as appropriate for the application.

An exemplary MRI safe biopsy system 10 includes a control module 12 that typically is placed outside of a shielded room containing an MRI machine (not shown) or at least spaced away to mitigate detrimental interaction with its strong magnetic field and/or sensitive radio frequency (RF) signal detection antennas. As described in U.S. Pat. No. 6,752,768, which is hereby incorporated by reference in its entirety, a range of preprogrammed functionality is incorporated into a control module 12 to assist in taking these tissue samples.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

The control module 12 controls and powers an MRI biopsy device 14 that is compatible for use in close proximity to the MRI machine. An example of an MRI biopsy device 14 is the afore-mentioned MAMMOTOME^{™} instrument. The MRI biopsy device 14 is accurately positioned by a single rail localization fixture 16 that is attached to a breast coil 18, which in turn supports a patient (not shown). Examples of commercially available breast coils 18 include the BIOPSY BREAST COIL MODEL BBC by INVIVO CORPORATION of Pewaukee, WI. A guidance assembly 20, and in particular a sleeve 22, advantageously attaches to the localization fixture 16 to increase imaging and therapeutic flexibility and accuracy in conjunction with selective use of the MRI biopsy device 14 at particular parts of the procedure. The guidance assembly 20 may include one or more obturators 24 with one depicted that seals the sleeve 22 during insertion and during subsequent portions of the procedure in which the MRI biopsy device 14 is not inserted therein. A depth stop 26 is provided for use with the localization fixture 16 to advantageously prevent over-insertion of the sleeve 22, inadvertent retraction of the sleeve 22 and/or to enhance accurate placement of the sleeve 22 to a desired location along the Z-Axis.

For convenience, herein a convention is used for locating a suspicious lesion by Cartesian coordinates within breast tissue referenced to the single rail localization fixture 16 and to thereafter position an instrument (e.g., sleeve 22) to this location without necessarily continuously imaging the region. A perforated barrier (described below) that is compressed along an outside (lateral) side of the breast, with respect to a medial plane of the chest of the patient, defines an X-Y plane, with the X-axis being vertical (sagittal) with respect to a standing patient and which corresponds to a left to right axis as viewed by a clinician facing the externally exposed portion of the single rail localization fixture 16. The fiducial device 11 includes a fiducial pointer 27 that may be positioned by the localization fixture 16 proximate to the patient's skin to define the origin of this plane. Perpendicular to this X-Y plane extending toward the medial side of the breast is the Z-axis, which typically corresponds to the orientation and depth of insertion of the MRI biopsy device 14, although it should be appreciated that variations may allow insertion at an angle to this Z-axis. Thus, for clarity, the term Z-axis may be used interchangeably with "axis of penetration", although the latter may or may not be orthogonal to the spatial coordinates used to locate an insertion point on the patient.

In use, contrast agent may be injected into the patient to enhance the imaging. The MRI compatible biopsy system 10 is prepared for use by removing a cap 28 from the fiducial pointer 27 and injecting an imageable material. The fiducial pointer 27 is then engaged to a fiducial holder 29. A cable management spool 30 is placed upon a cable management attachment saddle 32 that projects from a side of the control module 12. Wound upon the cable management spool 30 is a paired electrical cable 34 and mechanical cable 36 for communicating control signals and cutter rotation/advancement motions respectively. In particular, electrical and mechanical cables 34, 36 each have one end connected to respective electrical and mechanical ports 40, 42 in the control module 12 and another end connected to a holster 44 that receives the MRI biopsy device 14. An MRI docking cup 46, which may hold the holster 44 when not in use, is hooked to the control module 12 by a docking station mounting bracket 48.

An interface lock box 50 mounted to a wall provides a tether 52 to a lockout port 54 on the control module 12. The tether 52 is advantageously uniquely terminated and of short length to preclude inadvertent positioning of the control module 12 too close to the MRI machine. An in-line enclosure 56 may advantageously register the tether 52, electrical cable 34 and mechanical cable 36 to their respective ports 54, 42, 44 on the control module 12. A remote keypad 58 may be distally connected to the electrical cable 34 to enhance clinician control of the MRI biopsy device 14, especially when controls on the MRI biopsy device 14 itself are not readily accessible after insertion into the localization fixture 16.

Vacuum assist is provided by a first vacuum line 60 that connects between the control module 12 and an outlet port 62 of a vacuum canister 64 that catches liquid and solid debris. A tubing kit 66 completes the pneumatic communication between the control module 12 and the MRI biopsy device 14. In particular, a second vacuum line 68 is connected to an inlet port 70 of the vacuum canister 64. The second vacuum line 68 divides into two vacuum lines 72, 74 that are attached to the MRI biopsy device 14. With the MRI biopsy device 14 installed in the holster 44, the control module 12 performs a functional check. Saline is manually injected into biopsy device 14 to serve as a lubricant and to assist in achieving a vacuum seal. The control module 12 actuates a cutter mechanism (not shown) in the MRI biopsy device 14, monitoring full travel.

The portion of the MRI safe biopsy system 10 used near the MRI machine is also assembled. The generally known breast coil 18 is placed upon a gantry of the MRI machine, along with other body support pads (not shown). The localization fixture 16 is attached within a recess on either lateral side of the breast coil 18 to access a patient's breast that is pendulously exposed therein and includes a horizontal medial plate 80, a reusable base assembly 82, a lateral assembly 84, and a positioning pedestal 86. The localization fixture 16 is also assembled with a disposable medial fence 90 and a lateral window (or perforated plate) 92.

The base assembly 82 is placed within a selected lateral recess of the breast coil 18. The medial fence 90 attaches to a medial edge of the moveable, medial plate 80, aligned vertically approximately along a longitudinal axis of the breast coil 18 under an inner edge of a selected breast aperture 94 that receives a patient's breast. With the patient thus positioned and the outer area of the breast sterilized, the lateral window 92 is downwardly slid into a three-sided frame guide 96 of the lateral assembly 84, which in turn is placed upon the base assembly 82. The base assembly 82 and lateral assembly 84 are moved with respect to one another along the Z-axis to compress the patient's breast between the medial fence 90 and the lateral window 92. A mechanism formed between the lateral assembly 84, base assembly 82, and medial plate 80 maintains this compression. The medial plate 80 can also be moved laterally to provide more compression on the breast if necessary.

The positioning pedestal 86 is slidably engaged along the X-axis of the lateral assembly 84 and defines a vertical guide for positioning a single targeting rail ("track") 98 at a selected Y-axis coordinate. The track 98 in turn provides an alignment guide for placing the fiducial holder 29 of the fiducial device 11. The gantry is advanced into the MRI machine bore to image the localization fixture 16 and breast tissue. The fiducial device 11 inserted through the lateral window 92 is located and designated as the origin of the X-Y-Z coordinates. Then a suspicious lesion is located within the image and a point thereon is selected to determine its location relative to the origin. It should be appreciated that orienting the X-Y-Z axis of an initial scan may be facilitated by having the lateral window 92 formed of an imageable or non-imageable material. When the window material in non-imageable, the indenting profile of the window 92 creates regularly spaced deformations on the breast that can be imaged. Thus, the window 92 presents an X-Y plane in addition to the origin point of the fiducial device. With the target location determined, the gantry is withdrawn from the MRI machine bore.

With the fiducial device 11 then removed, the track 98 serves as an axis of penetration guide along the Z-axis for positioning the depth stop 26 and the holster 44 at a desired Z-axis coordinate. The depth stop 26 is latched onto the track 98. Thereafter, a marking instrument (not shown) may be inserted through the depth stop 26 to mark the insertion point on the breast. Alternatively, the fiducial pointer 27 may advantageously include a shaped distal end that temporarily dents or scores the breast tissue. Thus, the fiducial device 11 may then be repositioned to correspond to the desired insertion point to render such a marking indication. Thereafter, the depth stop 26 is moved out of the way. Anesthesia is injected superficially, followed by a scoring cut at the marked location and a subsequent injection of anesthesia more deeply into the scored cut. The depth stop 26 is then repositioned on the track 98 to the desired Z-axis coordinate reference.

The obturator 24 is inserted into the sleeve 22 and may be positioned to close any apertures of the sleeve 22 (side and/or distal end) to present a closed surface to the breast tissue. The obturator may also be shaped or formed to enhance the visibility of the aperture location. One or the other of the obturator 24 and sleeve 22 presents a sharp tip (not shown) to penetrate breast tissue. For instance, if using a sleeve 22 having an open end, an obturator may provide a sharp tip.

The obturator 24 is inserted into the sleeve 22 and the combination is guided by the track 98 to a proper orientation until an accurate depth is reached as set by the depth stop 26. Once fully inserted, the depth stop 26 prevents over-insertion. The sleeve 22 advantageously latches to the track 98 and/or the depth stop 26 to prevent inadvertent retraction, such as when the obturator 24 is withdrawn, and pressure is received from the breast tissue or later when a probe 100 of the MRI biopsy device 14 is withdrawn from the sleeve 22.

The gantry is moved into the MRI machine bore and the patient is imaged again to confirm placement of the sleeve 22 with respect to the suspicious lesion by locating a shape of a sleeve side aperture 102 that corresponds to a probe side aperture 104 of the probe 100 when inserted for subsequent biopsy samples.

The patient is removed from the MRI machine by retracting the gantry and the holstered MRI biopsy device 14 is brought to the localization fixture 16. A protective cap (not shown) is removed from the probe 100 of the MRI biopsy device 14 and the obturator 24 is removed from the sleeve 22. Features of the sleeve 22 and probe 100 may advantageously visually and mechanically orient a probe side aperture 104 of the probe 100 with the sleeve side aperture 102, as well as forming a gas seal. Advantageously, the holster 44 and/or the probe 100 may latch onto the track 98 or sleeve 22 to confirm full insertion and prevent over-insertion and inadvertent retraction. The holster 44 allows an MRI biopsy device 14 intended for handheld use to have sufficient support in its attachment to the localization fixture 16 to accurately maintain its position and to avoid or minimize loads carried by the probe 100.

Thereafter, the MRI compatible biopsy system 10 may take tissue samples by activating a cutter mechanism in conjunction with vacuum assist, withdrawing the cutter and withdrawing a tissue sample, the latter perhaps also with vacuum assist. The probe 100 / sleeve 22 combination are capable of manual, or perhaps automatic, rotation to a desired angle with respect to their longitudinal axis for additional samples or additional samples may be taken at the current orientation by further resorting to vacuum assist. The cutter is then advanced to close the probe side aperture 104 and the holster 44 is withdrawn from the localization fixture 16, thereby removing the probe 100 from the sleeve 22.

Additional steps or combinations of steps may be performed at this point such as using the probe 100, a specialized obturator 24 (e.g., stylet), or merely the sleeve 22 to guide various agents to the surgical site of the biopsy. Examples include draining fluids, inserting anesthetic agents, inserting haemostatic agents, insufflating with pneumatic pressure and inserting a marker for subsequently locating the site of the biopsy, or other diagnostic or therapeutic procedures.

The patient is then typically drawn back into the MRI machine bore for reimaging to confirm removal of at least a portion of the suspicious lesion and for possible placement of an inserted imageable marker. During this reimaging, the sleeve 22 is sealed with the obturator or stylet 24. Thereafter, the localization fixture 16 is removed, the patient bandaged and removed from the gantry, and the disposable portions of the MRI compatible biopsy system 10 disposed of as medical waste, perhaps including the fiducial device 11.

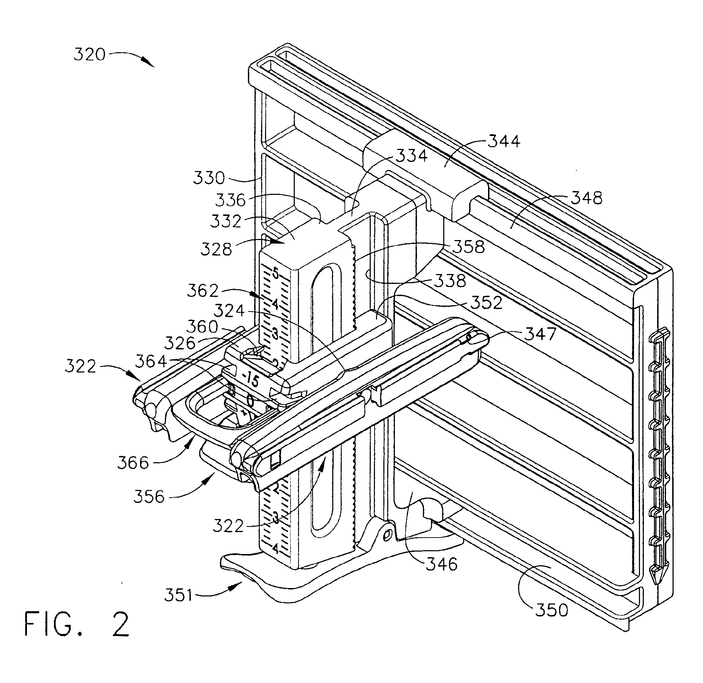

In FIG. 2, a lateral fence supported pedestal 320 provides an alternative support for spatially positioning a primary targeting rail 322 that in turn guides placement of the fiducial device 11 (FIG. 1), insertion of the sleeve 22 (FIG. 1) or other piercing biopsy devices (not shown). The primary targeting rail 322 includes an attachment axle 324 that receives in either a left or right side axle hub (not shown) of a (Y-axis) height yoke 326 that is vertically adjustable upon a pedestal 328, that in turn is laterally adjustable upon a lateral fence 330, which may be inserted in place of the medial plate 90 for accessing medially. The pedestal 328 includes a proximal upright rectangular column 332 with a thinner wall 334 projecting from its distal side that flares laterally outward (defining left and right vertical rectangular slots 336, 338) as part of a bracket 340 with top and bottom hanger arms 344, 346 that slide laterally respectively on a top track 348 and a bottom track 350 formed in the lateral fence 330. A lateral (X-axis) adjustment lever 351 may be raised to lift the pedestal 328 and thus the hanger arms 344, 346 out of engagement to the tracks 348, 350 as the lateral adjustment lever 351 is repositioned to the left or right to a desired location with reference to a lateral measurement guide (not shown).

The height yoke 326 is a rectangular cuff interrupted in a mid-portion of a distal side to form locking left and right hands 352 respectively which ride vertically in the left and right vertical rectangular slots 336. The locking left and right hands 352 have respective ridged proximal surfaces (not shown) that are selectively drawn proximally into locking engagement by a height locking lever 356 with a ridged surface 358 on a proximal side of each vertical rectangular slot 336. Lifting the height locking lever 356 unlocks the height yoke 326 for height adjustment. The proximal top surface of the height yoke 326 serves as a sight 360 to read a height measurement scale 362 presented on a proximal surface of the pedestal 328. Raising the height locking lever 356 takes the height yoke 326 out of locking engagement to the pedestal 328 as the height yoke 326 is vertically repositioned.

Symmetrical mounting provisions for the primary targeting rail 322 allow for use on either side of pedestal 328 so that full access may be made to the lateral fence 330. The attachment axle 324 allows rotation so that an axis of penetration may include an upward or downward trajectory. In the illustrative version, proximal corners of the height yoke 326 include angle detents 364 (e.g., -15°, 0°, +15°) that are selectable by an angle lock lever 366. The primary targeting rail 322 includes a distal detent 347 that serves as a home reference for the fiducial holder 29 (FIG. 1).

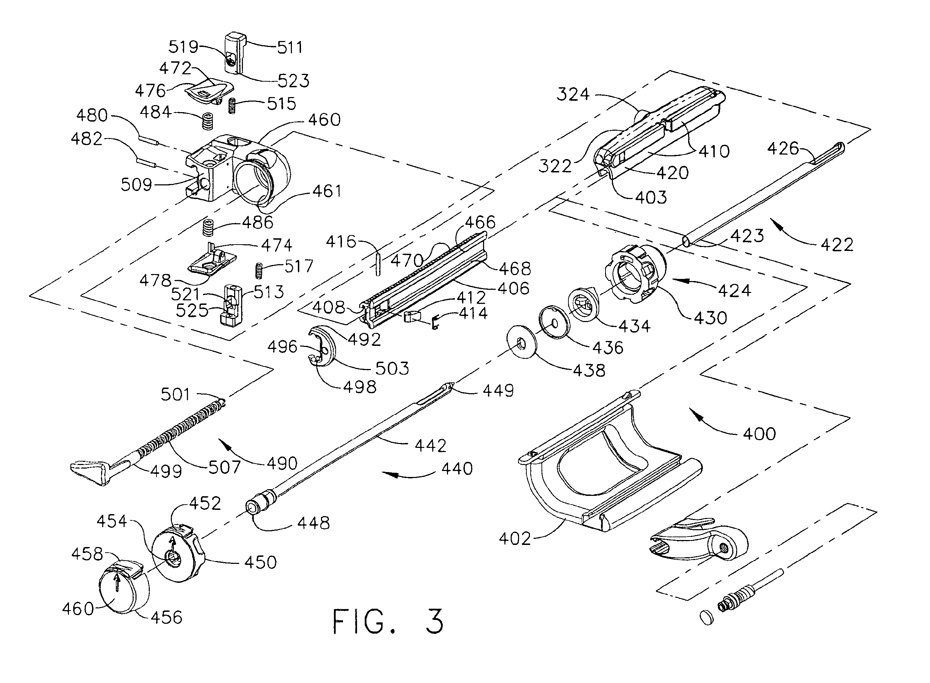

In FIG. 3, an alternative guidance assembly 400, that may be attached to the lateral fence supported pedestal 320 of FIG. 2, includes a cradle 402 that engages a bottom channel 403 of the primary targeting rail 322. To provide additional guidance to the MRI biopsy device 14 (FIG. 1), a secondary targeting rail 406 includes a lateral channel 408 that is guided along a longitudinal guide tab 410 of the primary targeting rail 322. When fully engaged thereon, a pawl 412 pivoting under urging of a pawl spring 414 about a vertical pawl pin 416 in a lateral window 418 proximally positioned in the secondary targeting rail 406 drops into a proximal detent 420 proximally positioned on the primary targeting rail 322. The pawl spring 414 may maintain the pawl 412 in a neutral position that serves in both assembly and later removal of the secondary targeting rail 406 or comprises a pair of opposing pawl springs (not shown) for that purpose.

A sleeve 422 includes a hollow shaft (or cannula) 423 that is proximally attached to a cylindrical hub 424 and has a lateral aperture 426 proximate to an open distal end 428. The cylindrical hub 424 has an exteriorly presented thumbwheel 430 for rotating the lateral aperture 426. The cylindrical hub 424 has an interior recess 432 that encompasses a duckbill seal 434, wiper seal 436 and a seal retainer 438 to provide a fluid seal when the shaft 423 is empty and for sealing to an inserted introducer obturator 440.

The introducer obturator 440 advantageously incorporates a number of components with corresponding features. A hollow shaft 442 includes a fluid lumen 444 that communicates between an imageable side notch 446 and a proximal port 448. The hollow shaft 442 is longitudinally sized to extend when fully engaging a piercing tip 449 out of the distal end 428 of the sleeve 422. An obturator handle 450 encompasses the proximal port 448 and includes a locking feature 452, which includes a visible angle indicator 454, that engages the sleeve thumbwheel 430 to ensure that the imageable side notch 446 is registered to the lateral aperture 426 in the sleeve 422. An obturator seal cap 456 may be engaged proximally into the obturator handle 450 to close the fluid lumen 444. The obturator seal cap 456 includes a locking or locating feature 458 that includes a visible angle indicator 460 that corresponds with the visible angle indicator 454 on the obturator thumbwheel cap 430. The obturator seal cap 456 may be fashioned from either a rigid, soft, or elastomeric material.

It should be appreciated that the internal diameter (lumen) of the hollow shaft 442 and the lateral aperture 426 of the introducer obturator 440 are advantageously dimensioned to permit precise deployment of a marker. The internal features of the obturator 440 may thus be equivalent to those present in a probe so that clinical flexibility is provided to deploy a marker with either cannula inserted into tissue. Alternatively, for probes that are not suited for marker deployment, the introducer obturator may provide a preferred approach to marker deployment.

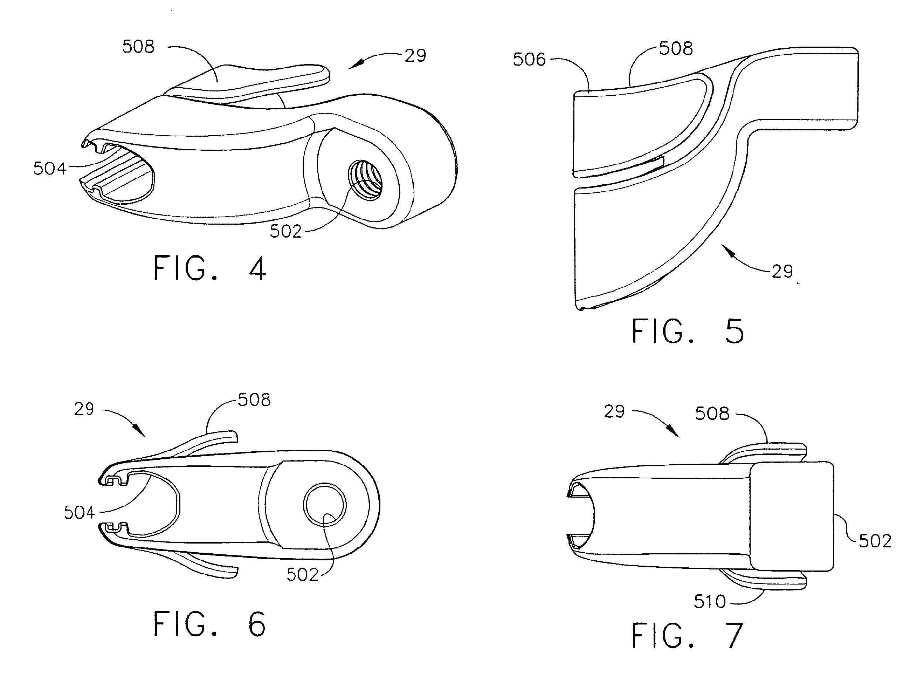

In FIGS. 4-7, the fiducial holder 29 includes an inner diameter (ID) threaded hub 502 that receives the fiducial pointer 27 (FIG. 1). A proximal channel arm 504 engages a primary targeting rail with a distal locking channel 506 that grips the primary targeting rail until a pair of release arms 508, 510 spread the distal locking channel 506.

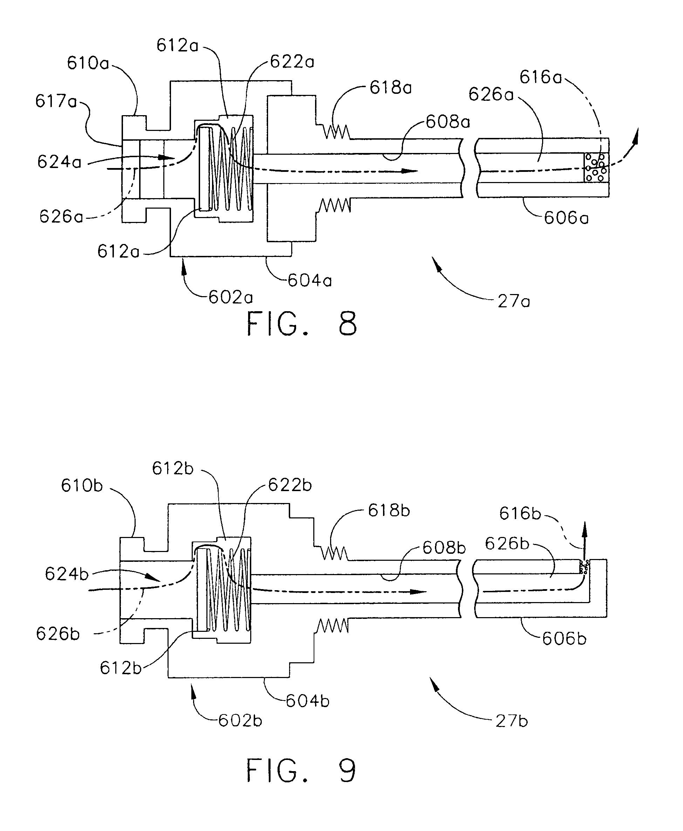

In FIG. 8, a short fiducial instrument 27a is an example of the fiducial device 27 in FIG. 1 used with a localization fixture 16 to locate a coordinate at an external point on the patient's skin. An optically transmissive (e.g., clear transparent, translucent, opaque) body 602a is assembled from a valve body 604a attached to a hollow snout 606a. Examples of such optically transmissive materials are a clear polycarbonate and thermoplastics. An imaging lumen 608a passes longitudinally from a proximal fill spout 610a proximally extending from the valve body 604a, through a one-way valve chamber 612a into an elongate cavity 614a in the hollow snout 606a whose distal end is partially sealed by a porous plug 616a. The porous plug may advantageously be formed of a hydrophilic material that is impregnated with or that accepts an MRI visible agent and/or ink. The latter may advantageously serve as a visible skin marking means. Examples of materials for the porous plug 616a include porous PTFE, porous polyethylene, porous polypropylene, polystyrene, and glass frit. External threads 618a on a proximal end of the hollow snout 606a allow for engagement to a holder, such as monocle or sleeve mount. In use, imageable fluid, such as but not limited to those materials described herein, are inserted into the proximal fill spout 610a by inserting a syringe needle (not shown) through a septum 617a that seals the proximal fill spout 610a, causing a seal 620a to unseat in the valve chamber 612a compressing valve spring 622a as the fluid enters the elongate chamber 614a as depicted by arrow 624a while air evacuates through porous plug 616a as depicted by arrow 626a. The end user continues to fill until evidently filled, as viewed through a clear polycarbonate body 602a, when resistance is felt while forcing in more fluid, when the fill spout 610a appears full, or when fluid begins to ooze through the porous plug 616a. It should be appreciated that a two-way valve may be included that would allow an over-pressure to release fluid or for a user to withdraw fluid. In addition, the septum 617a may suffice to hold fluid in the short instrument 27a without the need for the illustrated valve. Placement of the porous plus 616a at the distal end of the hollow snout 606a would lend itself to evacuating non-imaging air by aiming the fiducial device 27a upward with the porous plug 616a near its zenith, which may be convenient due to similar filling of syringes from a septum-closed bottle.

In FIG. 9, a long fiducial instrument 27b is an example of an imaging obturator or stylet or alternate features for a fiducial used external to the patient. Although not shown in FIG. 9 for clarity, a second open lumen may be included for inserting a tool. A piercing tip may also be included for use as an introducer obturator with an open ended sleeve (not shown in FIG. 9). A clear polycarbonate body 602b has an integral valve body portion 604b formed with a hollow snout portion 606b. An imaging lumen 608b passes longitudinally from a proximal pipe fitting (e.g., leur) 610b proximally extending from the valve body portion 604b, through a one-way valve chamber 612b into an elongate cavity 614b in the hollow snout 606b whose distal end is partially sealed by a small vent hole 616b that exits out of a lateral surface. Positioning of the small vent hole 616b lends itself to excluding non-imaging air from the distal end by pointing the fiducial device 27b slightly upward with the small vent hole 616b towards its zenith, which may be convenient in combination with the proximal pipe fitting 610b. Use of a pipe fitting, such as a leur fitting, 610b advantageously allows well-understood filling procedures to maintain sterility of the fiducial instrument 27b. External threads 618b on a proximal end of the hollow snout 606b allow for engagement to a holder, such as the sleeve hub 224 or the fiducial holder 29.

It should be appreciated with the benefit of the present disclosure that the proximal fill spout 610a and proximal pipe fitting 612 in combination with a porous plug opening 616a or open vent 616a at a distal end or near a distal end advantageously simplify filling of the fiducial instrument 27a, 27b without leaving air bubbles in the imaging lumen 608a, 608b that may impair the imageability of the fiducial instrument 27a, 27b. For instance, inserting a syringe needle down into a closed cavity of a container and slowly and carefully injecting liquids without forming bubbles may be difficult. For instance, after closing the opening in such a single-opening container, bubbles at the top may be allowed to propagate to the distal end when rotated upward for use. By contrast, the afore-mentioned fiducial instruments 27a, 27b tend to be fully filled with liquid and tend to capture any bubbles remaining in the proximal portion in one-way valve chamber 612a, 612b. In addition, certain versions of the fiducial instrument 27b may be filled with a syringe or similar fluid handling device without the necessity of a sharp object such as a needle. Thereby, personnel hazards due to inadvertent needle sticks and the requirement for disposal of a hazardous medical waste is mitigated.

In use, imageable fluid, such as but not limited to those materials described herein, are inserted into the proximal pipe fitting 610b, causing a seal 620b to unseat in the valve chamber 612b compressing closure valve spring 622b as the fluid enters the elongate chamber 614b as depicted by arrow 624b while air evacuates through vent hole 616b as depicted by arrow 626b. After filling, surface tension of the liquid prevents loss of fluid through the vent hole 616b.

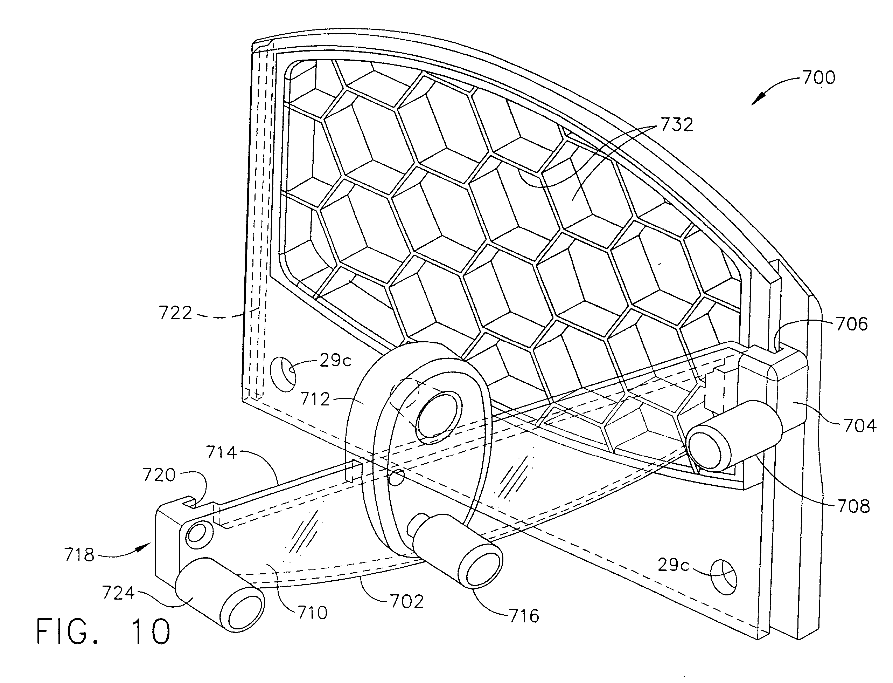

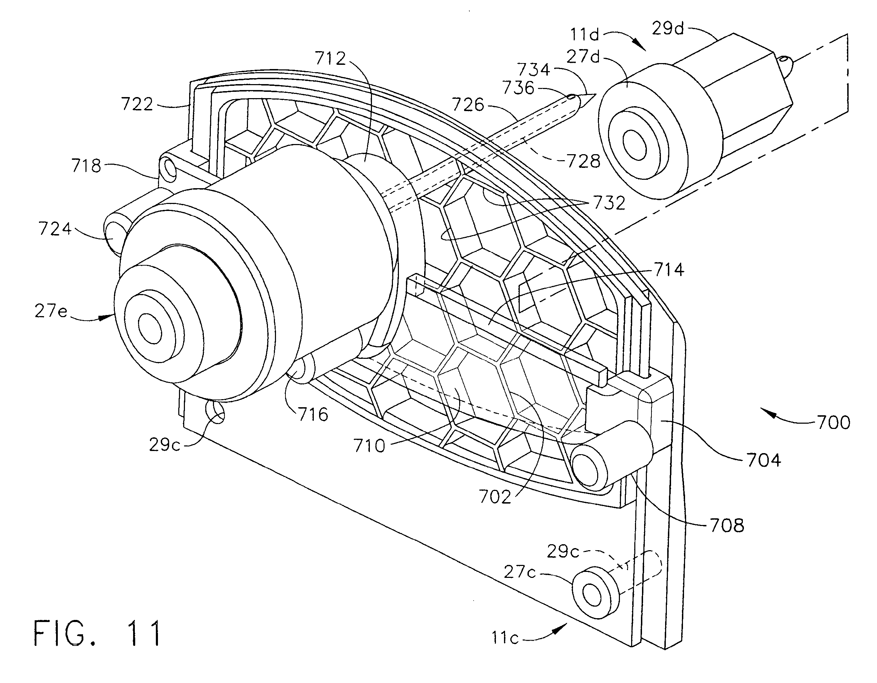

In FIGS. 10-11, a honeycomb lateral plate 700 with an integral distal targeting fixture 702 may be used with the MRI breast coil 18 and portions of a localization fixture 16 that compress the patient's breast. The integral distal targeting fixture 702 includes a vertically sliding, door hinged attachment 704 to a right-side channel 706 of the lateral plate 702. A right adjustment screw 708 locks the vertically sliding, door hinged attachment 704 to a particular vertical (Y) coordinate. A horizontal arm 710 of the integral distal targeting fixture 702 includes a reticule 712 that is horizontally slidingly engaged to a top track 714 and locks at a selected lateral (X) location by a middle locking screw 716. At a leftmost end of the horizontal arm 710, a latching mechanism 718 is formed by a grooved end 720 that engages a left-side vertical channel 722 of the lateral plate 700 that is held in position by a left locking screw 724, as shown in FIG. 11, which also shows a sleeve 726 inserted through the reticule 712. It should be appreciated that the sleeve 726 may interface the MRI biopsy device 14 (not shown in FIGS. 10-11).

With particular reference to FIG. 11, an illustrative comer-mounted fiducial device 11c having a short fiducial pointer 27c that may be inserted into a dedicated fiducial port 29c that serves as an integral fiducial holder is formed in a peripheral portion of the lateral plate 700. Thereby, a particular coordinate on the lateral plate 700 may be identified in a subsequent image. Alternatively or in addition, a grid-mounted fiducial device 11d may be formed by a short fiducial pointer 27d with a grid-shaped fiducial holder 29d that is sized for directed mounting into one of the openings in the lateral plate 700, which in the illustrative version comprises a hexagonal-shaped opening 732. It should be appreciated that other grid-shapes other than honeycombed may be used, such as round holes, square holes, elongate rectangular slots, etc. Thereby, additional clinical flexibility is realized by laterally positioning the fiducial device 11d closer to an area of interest, yet still be identifiable to a particular coordinate relative to the openings 732 available on the lateral plate 700. In addition, the length of the fiducial pointer 27d may be selected such that the skin of the patient is depressed slightly, forming an imageable dent with a contrast between skin, the structural material of the fiducial pointer 27d and any imageable material contained by the fiducial pointer 27d. Alternatively or in addition to the short fiducial pointers 27c, 27d, a long fiducial pointer 27e may be inserted into tissue, such as by serving as the obturator 728. To that end, a piercing tip 734 extends out of the sleeve 726 with a vent 736 positioned proximal to the piercing tip 734.

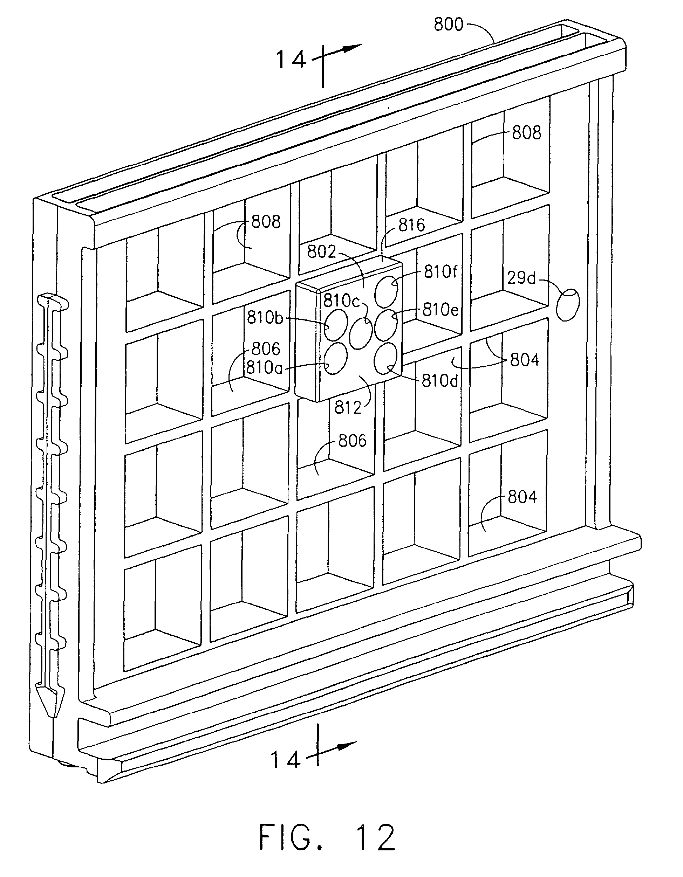

In FIG. 12, a grid lateral plate 800 may be downwardly inserted into the three-sided frame guide 96 of the lateral assembly 84, which in turn is placed upon the base assembly 82 of the localization fixture 16 of FIG. 1. Unlike the lateral fence that merely compresses a lateral surface of the patient's breast, the grid lateral window 800 in conjunction with a selectably inserted guide device, depicted as a guide cube 802, serves to localize and to guide an inserted biopsy sleeve, probe, or fiducial device. When performing a biopsy in the MRI environment, the patient is lying on the breast coil 18 (FIG. 1), and the pendulantly hanging breast is localized in part by the compression of the grid lateral window 800. The guide cube 802 is inserted into a selected circularly symmetric (e.g., square) aperture 804 defined by a plurality of horizontal bars 806 intersecting with a plurality of vertical bars 808.

In FIGS. 12-16, a fiducial instrument similar to the fiducial instruments 27a, 27b of FIGS. 8, 9 may be inserted into a dedicated fiducial port 29d formed in the grid lateral plate 800 (FIG. 12) or into one of a plurality of guide holes, depicted as horizontal holes 810a-810f that pass from a proximal face 812 to a distal face 814. An interference feature grounds the guide cube 802 against the grid lateral plate 800 to prevent passing on through to the distal side, which in the illustrative version is depicted as an increased width and height of a proximal hat portion 816 that abuts the edges of a selected square aperture 804.

Selection of an aperture 804 provides a coarse positioning in two coordinate planes (e.g., X-Y). Selection of one of the horizontal holes 810a-810f in the placed guide cube 802 further refines the coordinate positioning. While applications consistent with aspects of the invention need not have circularly symmetric apertures, an advantage thus provided is that positioning may be further refined by selectively rotating the guide cube 802 prior to insertion into the aperture 804 into one of four rotated positions. Thus, each horizontal hole 810a-810f is positionable in one of four positions within the aperture 804. Varying a vertical and lateral offset for each horizontal hole 810-810f may thus achieve access to as many as four times as many unique locations within each aperture 804 as the number of horizontal holes 810a-810f provided.

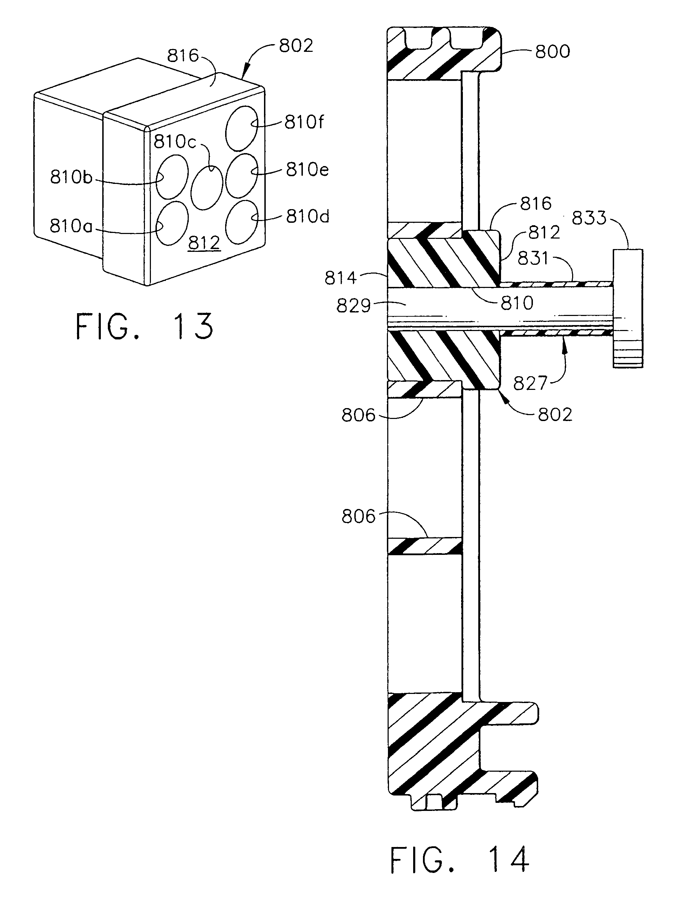

In FIG. 14, the guide cube 802 has been inserted into an aperture 804 of the grid lateral plate 800 until seated against the proximal hat portion 816. A fiducial instrument 827 has an elongate snout 829 sized for insertion into a horizontal hole 810 of the guide cube 802 until a sheath 831 on a proximal portion of the elongate snout 829 that is larger in diameter than the horizontal hole 810 prevents further insertion. A widened head 833 at a proximal end of the elongate snout 829 serves as a thumb push and gripping feature for retraction.

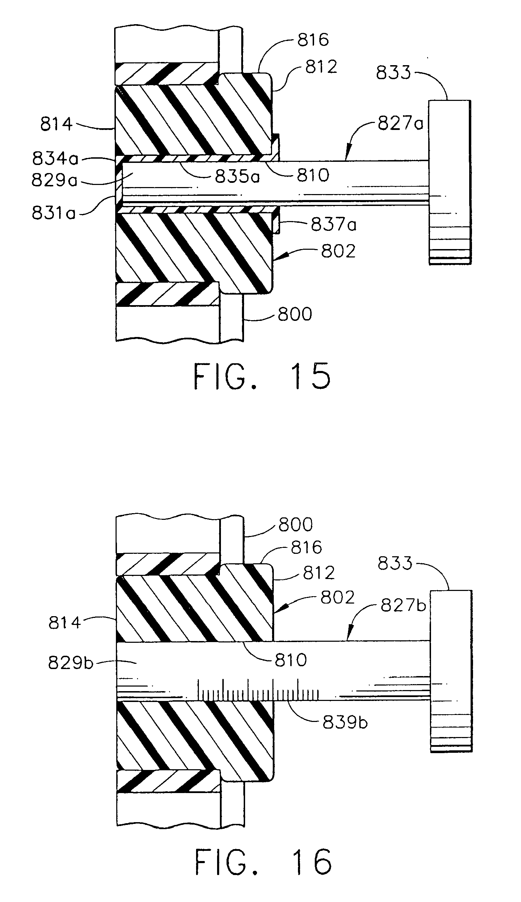

In FIG. 15, a sheath insert 831a, as an alternative to the sheath 831, encompasses a distal portion of a reduced diameter elongate snout 829a of a fiducial instrument 827a. The sheath insert 831a has a closed distal end 834a and cylindrical portion 835a sized to closely encompass the elongate snout 829 with both the sheath insert 831a and elongate snout 829a sized for insertion into the horizontal hole 810 until a proximal laterally flared end 837a of the sheath insert 831a abuts the proximal face 812 of the guide cube 802.

In FIG. 16, a further alternative fiducial instrument 827b has an elongate snout 829b sized for insertion into the horizontal hole 810 of the guide cube 802. Instead of a physical blocking feature that prevents over-insertion, measurement indicia 839 inscribed on the elongate snout 829b allow user control of the depth of insertion. For example, the distal end of the fiducial instrument 827b may dent the skin of the patient slightly to provide an imageable return outside of the plane of the grid lateral window 800 or tissue may be allowed to expand into the horizontal hole 810 against the imageable elongate snout 827b.

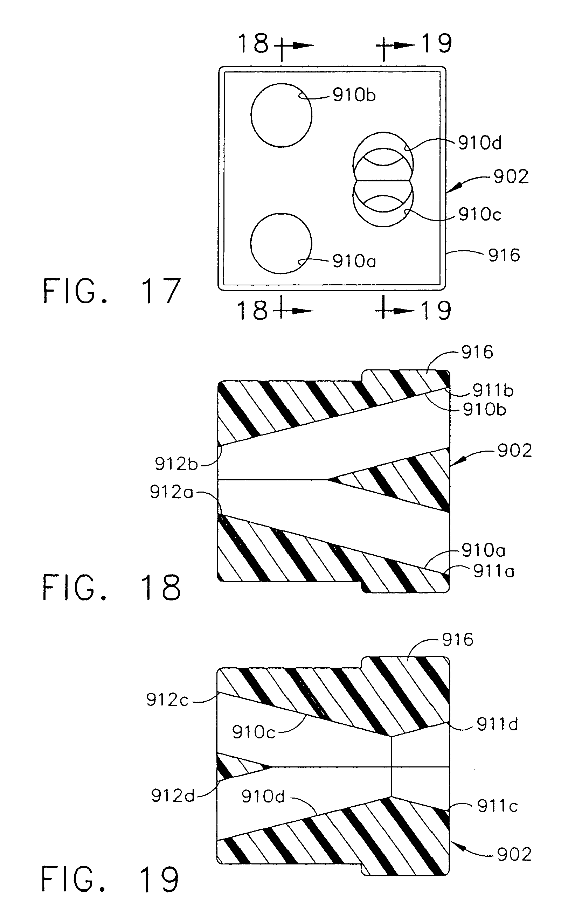

In FIGS. 17-19, an alternative guide cube 902 for the grid lateral plate 800 includes a proximal hat portion 916 for seating against a selected aperture 804 (FIG. 2). The guide holes are depicted as a first pair of converging angled through holes 910a, 910b having outwardly spaced proximal openings 911a, 911b (FIG. 18), respectively, that communicate with partially intersecting distal openings 912a, 912b, respectively. The guide holes are also depicted as a second pair of diverging angled through holes 910c, 910d having partially intersecting proximal openings 911c, 911d, respectively, that communicate with outwardly spaced distal openings 912c, 912d.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, other imaging modalities may benefit from aspects of the present invention.

For another example, a fiducial device may include a single structure that includes locking features and a fluid reservoir for imageable fluid.

For an additional example, a guide cube for insertion into a grid lateral plate may include an embedded fiducial device, either permanently sealed or perhaps accessible via a septum or pipe fitting (e.g., Leur fitting).

## Claims

1. An apparatus for performing a minimally invasive medical procedure with reference to a diagnostic image taken of a patient's breast compressed between a medial compression member and a lateral compression member having a biopsy probe support positionable relative to the lateral compression member, the apparatus comprising:
a housing formed of a magnetic resonance imaging (MRI) compatible material defining an internal cavity;
a port formed in the housing communicating with the internal cavity and operatively configured to receive MRI imageable material; and
a vent formed in the housing for allowing air evacuation during filling.

2. The apparatus of claim 1, wherein the port comprises a septum.

3. The apparatus of claim 1, wherein the port comprises a one-way valve.

4. The apparatus of claim 1, wherein the port comprises a two-way valve.

5. The apparatus of claim 1, wherein the port comprises a leur fitting.

6. The apparatus of claim 1, wherein the housing includes an elongate portion and a proximal portion including an engagement mechanism operatively configured for engagement to the biopsy probe support.

7. The apparatus of claim 1, wherein the housing comprises clear polycarbonate.

8. The apparatus of claim 1, wherein the housing comprises an optically transmissive thermoplastic.

9. The apparatus of claim 1, wherein the vent comprises a small hole.

10. The apparatus of claim 1, wherein the vent comprises a porous plug.

11. An apparatus for performing a biopsy with reference to a diagnostic image taken of a patient's breast, the apparatus comprising:
a medial compression member;
a lateral compression member moved relative to the medial compression member to compress and locate the patient's breast;
a pedestal member positionably coupled to the lateral compression member for locating a lateral coordinate; and
a targeting rail positionably coupled to the pedestal for locating a vertical coordinate and including a biopsy guide defining an angle of penetration.

12. An apparatus for performing a biopsy with reference to a diagnostic image taken of a patient's breast, the apparatus comprising:
a first compression member positionable on a selected side of the patient's breast;
a second compression member positionable on an opposite side of the patient's breast and moved relative to the first compression member to compress and locate the patient's breast;
an opening formed in the first compression member; and
a fiducial container fillable with an imageable fluid and sized for insertion into the opening in the first compression member.
